# EUROPEAN PATENT APPLICATION

(11) **EP 3 482 703 A1**
(43) Date of publication of application: **15.05.2019**
(21) Application number: 17201173.6
(22) Date of filing: 10.11.2017
(51) Int. Cl.: A61B 17/70

(54) **DISTRACTION AND COMPRESSION ASSEMBLY, IN PARTICULAR FOR BONE SURGERY**

(71) Applicant: Biedermann Technologies GmbH & Co. KG, 78166 Donaueschingen (DE)
(72) Inventor: BIEDERMANN, Timo, 78647 Trossingen (DE); DOLD, Kevin, 72351 Geislingen (DE)
(74) Representative: Prüfer & Partner mbB Patentanwälte · Rechtsanwälte

(57) **Abstract**

A distraction and/or compression assembly, in particular for use in bone surgery, is provided including a first clamping member (1, 101) configured to engage a a first bone anchor (10), the first bone anchor comprising a head (12) and a neck (13) adjacent to the head (12), a second clamping member (1', 101') configured to engage a second bone anchor (10'), wherein the second bone anchor comprises a head (12) and an neck (13) adjacent to the head (12), a connection member (100) configured to connect the first clamping member (1, 101) and the second clamping member (1', 101'); wherein at least the first clamping member (1, 101) comprises a clamping element (20, 20') that can assume a clamping position in which the clamping element (20, 20') is configured to laterally enclose an head (12) of the bone anchor (10, 10') and to exert pressure onto the neck (13) from one side.

## Description

The invention relates to a distraction and/or compression assembly, in particular for use in bone surgery.

Distraction and compression steps during spinal surgery are well-known in the art. For example, in the case of single or multilevel discectomies, cages, pedicle screws and rods are often used for providing stability to the spinal segments. In order to remove the intervertebral disk and to insert a cage into the intervertebral space the vertebrae are distracted. This is accomplished by using, for example, distraction pliers that engage two adjacent pedicle screws along a rod captured therein and spread them apart from each other. Thereby, the intervertebral space is enlarged. During distraction considerable loads may act onto the vertebrae. In some constellations unwanted loads may be detrimental to the anchoring of the pedicle screws in the bone.

In minimally invasive surgery (MIS) only small incisions are made and therefore, the available space for the surgical manipulations is reduced. This is also the case in other fields of spinal surgery, such as cervical spinal surgery or pediatric spinal surgery.

Devices, systems and methods for minimally open also pedicle spine surgery are known, for example from US 8,979,749 B2.

It is the object of the invention to provide a distraction and/or compression assembly, in particular for bone surgery, more particularly for spinal surgery, that is improved in terms of utilization of the available space at the implant site and/or in terms of reducing unwanted loads acting onto the bone anchors and the bone.

The object is solved by a distraction and/or compression assembly according to claim 1. Further developments are given in the dependent claims.

The assembly can be used in connection with bottom loading polyaxial pedicle screws or, more generally, bottom loading polyaxial bone anchors. Such bottom loading polyaxial pedicle screws or bone anchors include a bone anchoring element that comprises a shank and a head with a spherical outer surface portion and a receiving part for polyaxially coupling the bone anchoring element to a spinal stabilization rod. In particular in MIS, the bone anchoring element may be first inserted into the bone before the receiving part is mounted thereon. The distraction and the compression assembly can be mounted in-situ onto an inserted bone anchoring element of such a bottom loading polyaxial pedicle screw or bone anchor. Moreover, the distraction and compression assembly includes clamping members that require less space compared to conventional polyaxial pedicle screws. Hence, with the assembly, less space is needed and therefore more space may be available for the instruments removing an intervertebral disc or inserting a cage or other another intervertebral implant. Also, the visibility for the surgeon might be improved.

At the same time, the assembly can be easily adapted to various angular positions of the bone anchoring elements the heads of which project out of the bone surface. The versatility of the assembly is therefore increased.

According to one aspect, the heads of the bone anchors are after mounting of the assembly and fixing the clamping members are still able to pivot relative to the clamping member to some extent and only to one side. By means of this, unwanted loads acting onto the bone anchors and in turn on the bone can be reduced or avoided.

The assembly can be used as an auxiliary tool for distraction and/or compression that is removed after the intervertebral implant has been inserted. After the intervertebral implant has been inserted, the assembly is replaced by the receiving parts of the polyaxial bone anchors and by the spinal stabilization rod.

Mounting and removing of the assembly during surgery can be performed quickly and in a simple manner. This saves time during surgery.

In an embodiment, the assembly can be selectively used as a distraction or as a compression assembly depending on the arrangement of the clamping members.

Further features and advantages of the invention will become apparent from the description of embodiments by means of the accompanying drawings.

In the drawings:
- Fig. 1: shows a perspective exploded view of the distraction and compression assembly according to a first embodiment.
- Fig. 2: shows a perspective view of the assembly of Fig. 1 in an assembled state.
- Fig. 3: shows a perspective view from the top of a clamping member of the assembly of Figs. 1 and 2.
- Fig. 4: shows a perspective view from the bottom of the clamping member of Fig. 3.
- Fig. 5: shows a bottom view of the clamping member of Figs. 3 and 4.
- Fig. 6: shows a top view of the clamping member of Figs. 3 to 5.
- Fig. 7: shows a cross-sectional view of the clamping member of Figs. 3 to 6 along line A-A in Fig. 6.
- Fig. 8: shows an outer perspective view of a clamping element of the assembly of Figs. 1 to 7.
- Fig. 9: shows an inner perspective view of the clamping element of Fig. 8.
- Fig. 10: shows a top view of the clamping element of Figs. 8 and 9.
- Fig. 11: shows a cross-sectional view of the clamping element of Figs. 8 to 10 along line B-B in Fig. 10.
- Figs. 12a to 12c: show cross-sectional views of steps of mounting the assembly and fixing the assembly to bone anchoring elements.
- Figs. 13a to 13b: show perspective views of mounting the assembly to the bone anchoring elements.
- Fig. 14a: shows a perspective view of a distraction step using the assembly of Figs. 1 to 13b.
- Fig. 14b: shows a cross sectional view of a distraction step using the assembly of Figs. 1 to 13b.
- Fig. 15: shows a perspective view of an embodiment of a compression assembly in the assembled state.
- Fig. 16: shows a perspective view of another embodiment of a distraction assembly.
- Fig. 17: shows a perspective view from the top of the distraction assembly of Fig. 16 together with a retractor system in use.

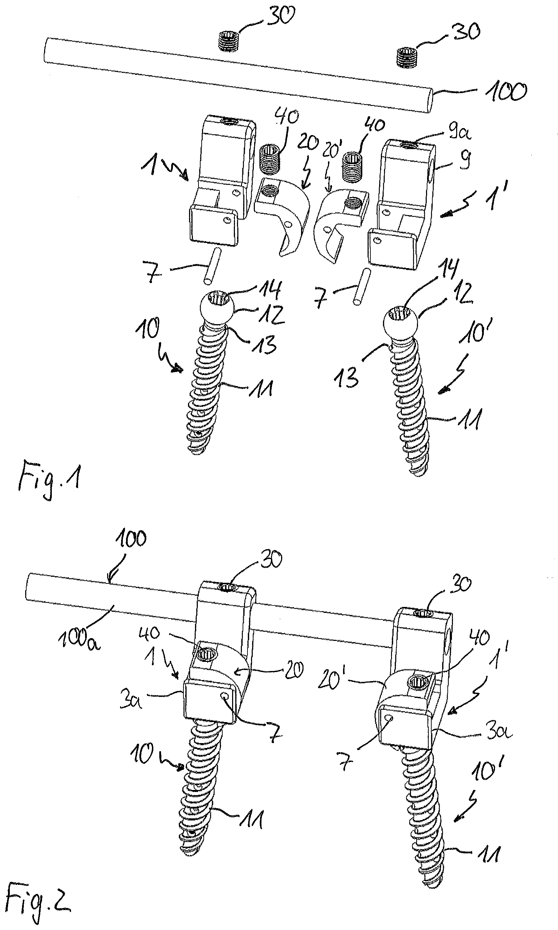

A first embodiment of a distraction assembly will be described referring to Fig. 1 to Fig. 14b. As shown in Figs. 1 and 2, the assembly includes two clamping members 1, 1' and a connection member in the form of a rod 100. The clamping members 1, 1' are configured to be connected to bone anchors 10, 10', respectively. At the same time, the clamping members 1, 1' are configured to be connected to the rod 100. The bone anchors 10, 10' in this embodiment are formed as bone screws, each having a shank 11 that is configured to be anchored in bone or in a vertebra and a head 12. The head 12 comprises a spherically-shaped outer surface portion. More in detail, the head 12 may be shaped as a segment of a sphere and arranged such with respect to the shank 11 that the diameter of the head 12 gradually decreases towards a neck portion 13 arranged between the head 12 and the shank 11. At a free end of the head 12 a recess 14 for engagement with a driver may be provided.

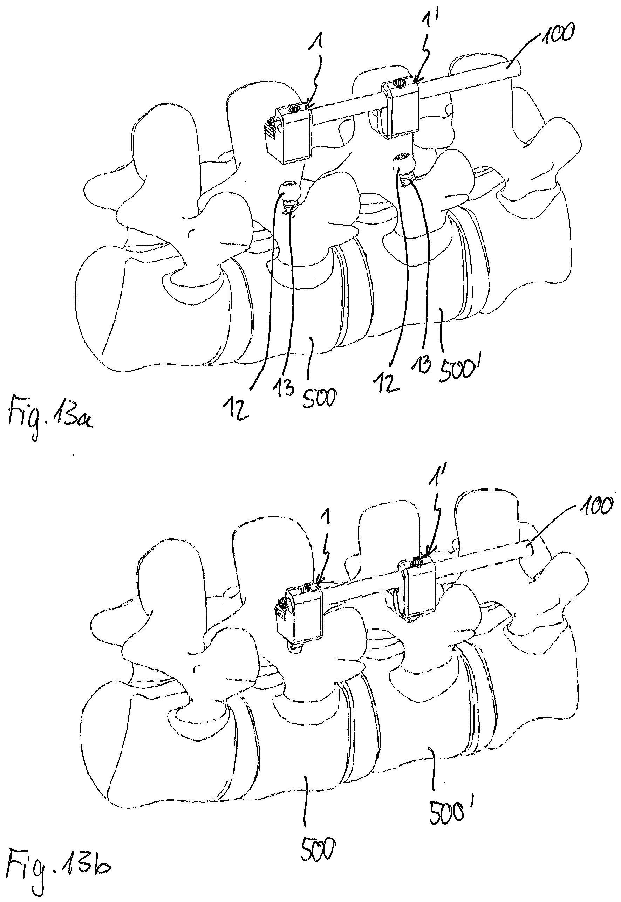

The rod 100 has a substantially cylindrical shape with a substantially constant diameter along its length. The length of the rod 100 may be such that at least one motion segment of the vertebral column, that means at least the distance from one vertebra to an adjacent vertebra, can be spanned. Preferably, the length of the rod is greater than the distance between two adjacent vertebrae in a region of the spinal column where the distraction and compression assembly is intended to be used. As depicted in Fig. 2, the clamping members 1, 1' are mounted onto the rod 100 with a distance between each other that approximately corresponds to the distance between two adjacent pedicles (see for example Fig. 13a and 13b). Moreover, the clamping members 1, 1' are mounted such that one end of the rod 100 is flush with one outer surface of one of the clamping members, in this example the clamping member 1', or does not project out of the surface. The other end of the rod 100 projects out of the corresponding outer surface of the other clamping member 1. The length of the rod 100 is selected such that the projecting portion 100a has a length sufficient for gripping and placing the assembly onto the bone screws 10, 10' as explained below.

The rod 100 may have a smooth surface to facilitate sliding or stepless movement of the clamping members 1, 1'. Alternatively, the rod surface may have an engagement structure that is configured to cooperate with another engagement structure, such as, for example ratchet means, to permit stepwise movement of the clamping members 1,1' relative to the rod 100.

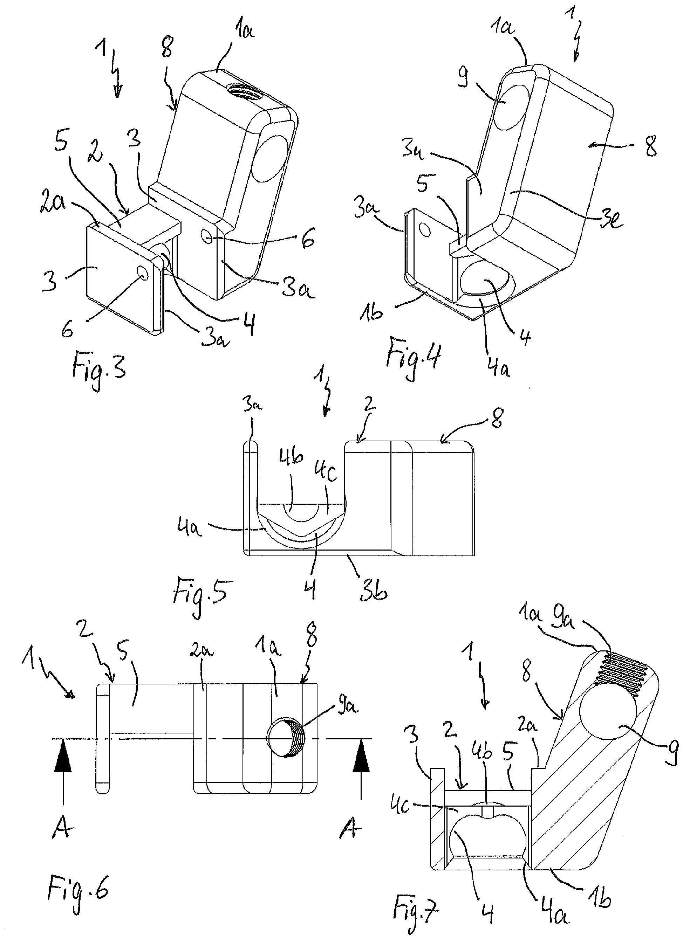

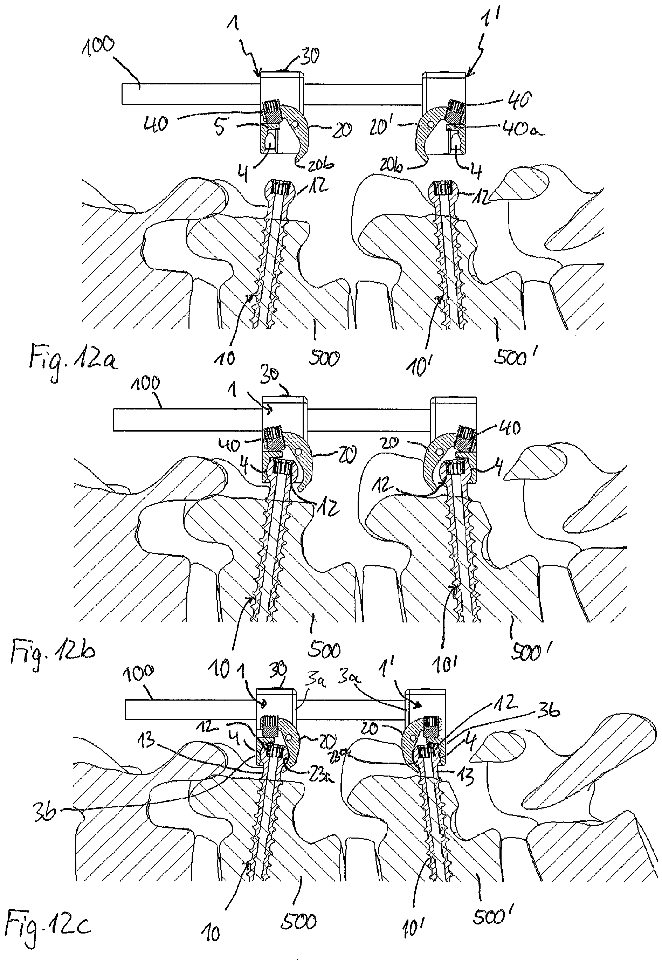

The clamping members will be described more in detail with additional reference to Figs. 3 to 7. As can be seen in Figs. 1 and 2, the clamping member 1, 1' are shaped and arranged mirror-symmetrical. In the following, it is referred to the clamping member 1 of Fig. 1. The clamping member 1 comprises a head receiving portion 2 that has in a top view a substantially rectangular contour and comprises two spaced apart sidewalls 3 a lower end of which defines a bottom side 1b of the clamping member 1. An upper side of the sidewalls 3 forms an upper side 2a of the head receiving portion 2. A front edge of the sidewalls 3 define a front side 3a of the clamping member 1. Opposite to the front side 3a the sidewalls 3 are connected at their rear ends to form a rear side 3b of the clamping member 1. Between the sidewalls 3 there is a head receiving recess 4 that is open towards the bottom end 1b and towards the front side 3a. The head receiving recess 4 forms a seat portion for the head 12. More in detail, the head receiving recess 4 has a substantially spherically- shaped portion that matches a shape of the outer surface portion of the head 12 of the bone anchor 10 (see Fig. 12c). The head receiving recess 4 may widen towards the bottom end 1b of the clamping member 1 through a taper 4a, as shown, for example, in Fig. 7. The taper 4a permits an inserted head 12 of the bone anchor and the neck 13 to pivot relative to the clamping member 1 in a direction towards the rear side 3b. Opposite to the bottom end 1b, the head receiving recess 4 is closed. More in detail, a top wall 5 is arranged on top of the head receiving recess 4 at a height position below the upper side 2a. The top wall 5 has a substantially rectangular contour and projects in a lateral direction beyond the head receiving recess 4, in the embodiment the top wall ends substantially approximately halfway between the front side 3a and the rear side 3b of the head receiving portion 2. An upper surface of the top wall 5 forms a support surface for a head locking member as explained below. A recessed portion 4b, 4c at a lower surface of the top wall 5 and around the head receiving recess 4 may provide space to allow an inserted head 12 to pivot.

In a region close to the front side 3a and to an upper edge 2a of the sidewalls 3 two opposite holes 6 are formed in the sidewalls 3. In the holes, a pin 7 (Fig. 1) is supported that serves as an axis of rotation for the clamping elements 10, 10'.

Adjacent to the head receiving portion 2 a rod receiving portion 8 is provided the sidewalls of which are flush with the front side 3a and the rear side 3b of the head receiving portion. An upper end 1a of the rod receiving portion forms an upper end of the clamping member 1 and a lower end of the rod receiving portion 8 is flush with the bottom side 1b of the head receiving portion 2. More specifically, the rod receiving portion 8 forms an upwardly extending arm that projects upward and slightly away from the head receiving portion 2, so that the connection to the rod 100 is laterally offset from the connection to the bone anchor. As can be seen in Fig. 7, the rod receiving portion 8 is inclined outward with respect to the head receiving portion 2. At a distance from the upper end 1a of the clamping member 1, the rod receiving portion 8 comprises a rod guiding recess 9. The rod guiding recess 9 may be a cylindrical through-hole that extends from the front side 3a to the rear side 3b. A diameter of the rod guiding recess 9 is such that the rod 100 can slide therein. At approximately the middle between the front side 3 a and the rear side 3b a threaded hole 9a is provided that extends from the upper end 1a into the rod guiding recess 9 for receiving a rod locking member 30 for locking the clamping member 1 to the rod 100 therein. The rod locking member 30 can be, for example, a set screw as shown in Figs. 1 and 2. The clamping member may have beveled edges 3e, in particular at the front side 3a

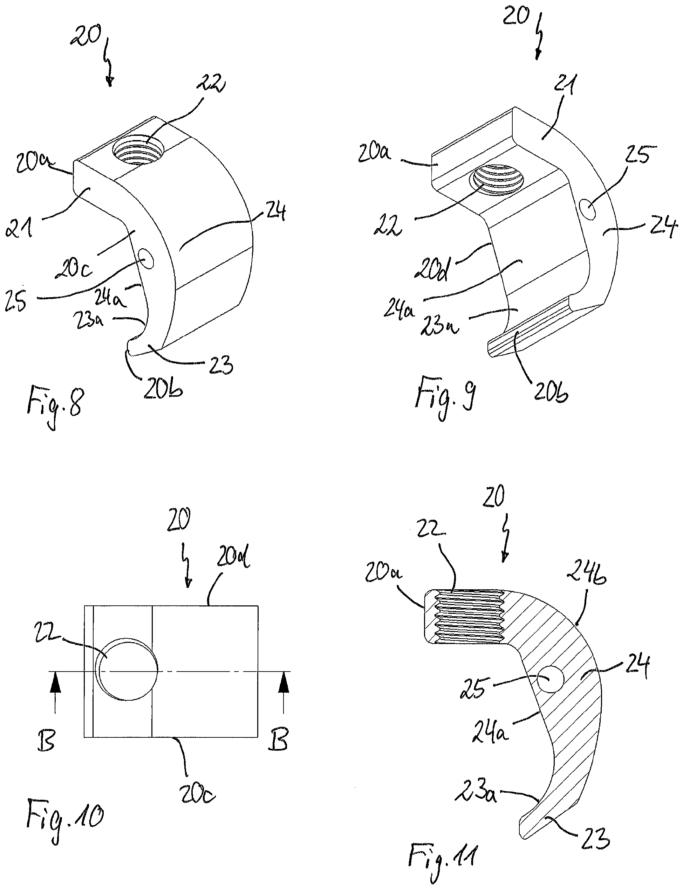

Turning now to Figs. 8 to 11, the clamping element 20 will be described. It shall be noted that the clamping element 20' is identical to the clamping element 20. The clamping element 20 has a hook-like shape with a width that is such that the clamping element 20 fits in a space between the sidewalls 3 of the head receiving portion, as depicted in Figs. 1 and 2. The clamping element 20 has a first or upper free end 20a and opposite thereto a second or lower free end 20b. A portion 21 adjacent to the upper free end 20a is substantially flat and comprises a threaded through-hole 22 substantially in the center thereof for receiving a head locking member 40 therein (see Figs. 1 and 2). An end portion 23 adjacent to or close to the lower free end 20b comprises a substantially cylinder segment-shaped inner surface portion 23a, a cylinder radius of which substantially corresponds to an outer contour of the head 12 of the bone anchor 10 (Fig. 12c). An intermediate portion 24 of the clamping member has a substantially flat inner surface portion 24a. Furthermore, a transverse through-hole 25 is provided in the intermediate portion 24 that extends in a transverse direction from one side 20c to the opposite side 20d of the clamping element 20. The through-hole 25 is configured to receive the pin 7 therein. An outer contour of the clamping element 24b in the intermediate portion 24 may be rounded, in particular cylinder segment-shaped. As can be seen in particular in Figs. 1, 2 and 12a to 12c, the size of the clamping element 20, 20' is such that when the clamping element 20, 20' is mounted to the clamping member 1, 1', respectively, it fits between the sidewalls 3 and extends slightly above the upper end 2a of the head receiving portion 2 and slightly outward from the front side 3a. Moreover, the size of the clamping element 20, 20' is such that in a clamped state as shown in Fig. 12c, the clamping member 20, 20' fits tightly around the head 12 of the bone anchor 1.

The head locking member 40 is formed as a set screw which preferably has a rounded bottom face 40a shown in particular in Figs. 12a to 12c. The rounded bottom surface 40a of the head locking member 40 abuts against the top wall 5 and is configured to pivot on the top wall 5 to some extent when the clamping member 20, 20' pivots around the pin 7.

The clamping elements 20, 20' each can assume an open position that means the inner surface 24a of the intermediate portion 24 is spaced apart from the head receiving recess 4 by a distance that is greater than a diameter of the head 12. As the clamping elements 20, 20' each form a lever that is rotatably supported by the pin 7, they can pivot to some extent around the pin 7. By tightening the head locking member 40, the clamping element 20, 20' rotates around the pin 7 until it assumes a closed position where the lower free end 20b can abut against the neck 13 or shank 11 of the bone anchor.

The distraction assembly may be pre-assembled. In the pre-assembled condition, the clamping members 1, 1' are already mounted onto the rod 100. i.e. the rod 100 is guided through the rod recess 9. The positions of the clamping members 1, 1' on the rod 100 are such that the rod 100 does not project out of a rear surface 3b of the clamping member 1' but projects out of the rear surface 3b of the clamping member 1. In the embodiment shown, the clamping member 1' is fixed to the rod 100 by means of the rod locking member 30. The other clamping member 1 is movable along the rod axis. This can be achieved by not tightening the rod locking member 30. The clamping members 1, 1' are oriented in such a manner on the rod 100 that the clamping elements 20, 20' are facing each other and also the front sides 3 a of the clamping members 1,1' are facing each other. Initially, a distance between the clamping members 1, 1' is selected such that the distance corresponds approximately to the distance of the heads 12 of the bone anchors 10, 10' in a direction along the length of the spinal column.

The bone anchors 10, 10', the clamping members 1, 1' and their elements including the clamping elements 20, 20' as well as the rod 100 may each be made of bio-compatible materials, for example of titanium or stainless steel, of a bio-compatible alloy, such as a NiTi-alloy, for example Nitinol, of magnesium or magnesium alloys, or from a bio-compatible plastic material, such as, for example, polyether ether ketone (PEEK) or poly-1-lactide acid (PLLA). The parts can all be made of the same material or of different materials.

Turning now to Figs. 12a to 12c, steps of using the assembly as a distraction assembly will be explained. First, as illustrated in Fig. 12a, the bone anchors 10, 10' have already been inserted into the bone, such as into the pedicles of adjacent vertebrae 500, 500'. The heads 12 and the necks 13 or at least a portion thereof project out of the bone surface. An orientation of the bone anchors 10, 10' with respect to the bone surface may not be perpendicular but angled, as depicted in the Figures.

The distraction assembly in the pre-assembled state is mounted onto the heads 12 of the bone anchors 1, 1' as shown in Fig. 12b. Thereby, the heads 12 enter the space between the clamping element 20, 20' and the head receiving recess 4 of each clamping member 1, 1', respectively, until the head 12 is supported at one side in the head receiving recess 4 as depicted in Fig. 12b.

Thereafter, as shown in Fig. 12c, by tightening the head locking member 40, the clamping elements 20, 20' are pivoted respectively to the closed position in which the lower free end 20b goes around a lower side of the head 12 until it abuts against the neck 13. A small gap may still be present between the inner surface 24a and 23 of the clamping elements 20, 20' and the head 12. However, as soon as the free lower end 20b of the clamping element 20, 20' goes around the head 12, the head 12 is secured to the clamping member 1, 1' and the clamping member 1, 1' cannot be removed without loosening the head locking member 40.

Finally, as also shown in Fig. 12c, the head locking member 40 is finally tightened so that the position of the clamping elements 20, 20' is fixed, respectively. In this position, the head 12 is laterally enclosed by the clamping member 20, 20' on one side of the head 12. As the clamping members 20, 20' each press only from one side onto the heads 12 and/or the necks (13), respectively, the heads 12 are not completely locked. As can be seen from Fig. 12c, the heads 12 of the bone anchors 10, 10' can still pivot with respect to the head receiving recess 4 towards the rear side 3b, while the lower free end 2b of the clamping members 20, 20' would block pivoting towards the front side 3 a.

Figs. 13a and 13b show perspective views of mounting the pre-assembled distraction assembly onto the heads 12 of inserted bone anchors 10, 10'. Once mounted onto the heads 12 of the bone anchors 10, 10' the assembly is ready for the distraction step.

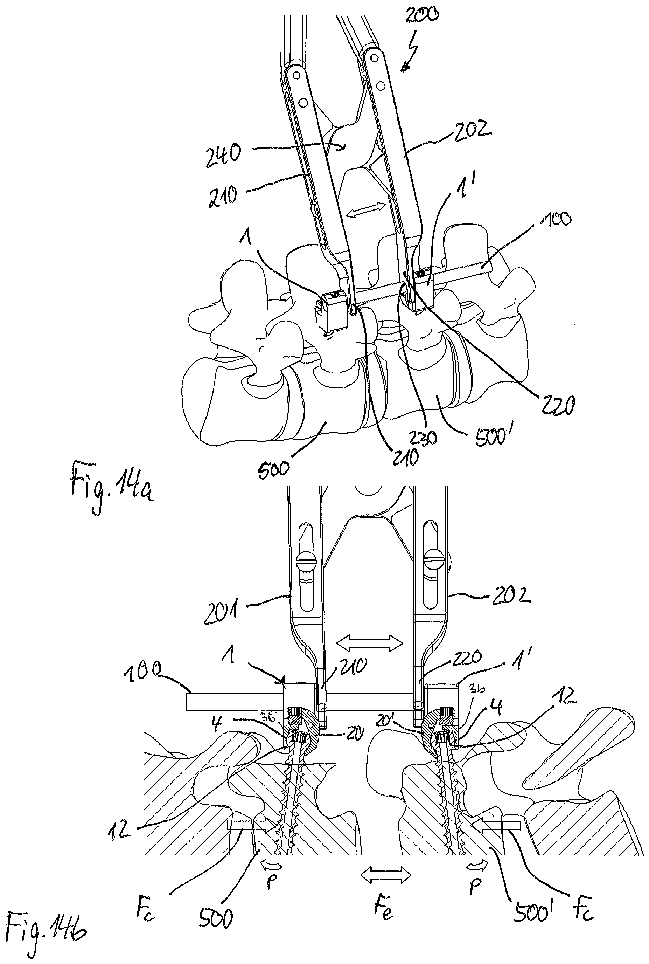

As illustrated in Fig. 14a, distraction pliers 200 are placed onto the rod 100. The distraction pliers 200 comprise two arms 201, 202 with front ends 210, 220, each having a recess 230 for placing the front ends 210, 220 over the rod 100. The arms 201, 202 are connected via a lever arrangement 240 such that compressing the handles of the arms 201, 202 (not shown) spreads the front ends 210, 220. As one arm 210 abuts against the fixed clamping member 1', the other arm 220 moves the movable other clamping member 1 away from the fixed clamping member 1'. As the clamping members are relatively small, enough space is available at the operation site for visibility and/or manipulations.

As depicted in Fig. 14b, during parallel distraction the adjacent vertebrae 500, 500' move apart from each other. Thereby the intervertebral space is enlarged. Once an appropriate enlargement has been achieved, the movable clamping member can be fixed with the rod locking member 30 to keep the enlargement. The pliers 200 may be removed. Then, discectomie, i.e. removal of the intervertebral disc, and insertion of a cage or another intervertebral implant can be carried out (not shown). When inserting a cage or another intervertebral implant, a force Fₑ (arrow Fig. 14b) may be generated that tends to further spread apart the vertebrae. This causes a counterforce F_{c} to act onto the shanks 11 of the bone anchors 10, 10'. As the free end 20b of the clamping element 20, 20' abut against the neck 13 of the bone anchor 10, 10', the head 12 cannot pivot in the direction towards the clamping elements 20, 20'. However, the head 12 is still able to pivot in the opposite direction towards the rear side 3b, which is shown by the curved arrows p in Fig. 14b. By means of this, unwanted loads acting onto the bone anchors and in turn on the vertebrae can be reduced or avoided.

Finally, the assembly of the clamping members 1,1' and the rod 100 is removed. To achieve this, the head locking members 40 are loosened so that the clamping elements 20, 20' are able to pivot so that the clamping members 1, 1' can be pulled away from the heads 12.

Thereafter, receiving parts of polyaxial bone anchors are mounted onto the heads 12 and a spinal stabilization rod that is different from the rod 100 is inserted into the receiver members. Lastly, the polyaxial receiving parts are fixed to the heads and to the rod to achieve the final stabilization.

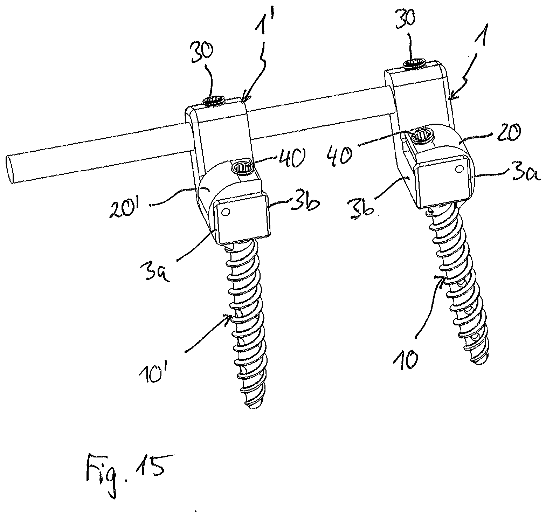

An embodiment of a compression assembly is shown in Fig. 15. The compression assembly comprises parts that are identical to the distraction assembly as shown in Figs. 1 to 14b. The parts are indicated with the same reference numerals and the description thereof is not repeated. In the compression assembly the clamping members 1, 1' are arranged on the rod 100 in such a manner that the rear surfaces 3b of each of the clamping members 1, 1' are facing towards each other. In turn, the clamping elements 20, 20' face away from each other. One of the clamping members 1, 1', in this embodiment the clamping member 1 is mounted to an end of the rod 100 and fixed thereon. The other clamping member 1' is slideable on the rod 100.

In use, the compression assembly is mounted on the bone anchors 10, 10' that have been inserted into a bone or a vertebra. Then, compression pliers (not shown) are used that engage the front side 3 a of the clamping members 1, 1' so that the clamping members can be moved towards each other, thereby compressing the intervertebral space. In a modification of the assembly, the clamping members 1, 1' are mounted onto the rod 100 in such a way that the rod 100 projects out of the front side 3a of each of the clamping members 1, 1' so that the compression pliers can engage a rod portion on both sides of the clamping members 1, 1'.

The distraction and compression assemblies described above may be assembled before use according to the desired step, i.e. distraction or compression, by orienting and mounting the clamping members 1, 1' in an appropriate manner. A modular system can be provided that comprises rods of different length and at least two clamping members 1, 1' as described above that can be assembled with the rod according to the needs.

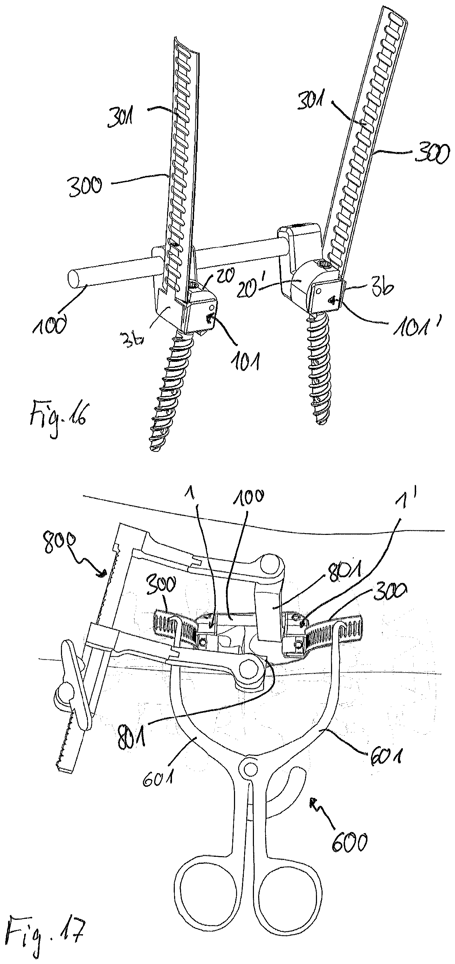

Next, another embodiment of a distraction assembly will be explained with respect to Figs. 16 and 17. In Fig. 16, the distraction assembly comprises modified clamping members 101, 101'. The clamping members 101, 101' differ from the previously described clamping members in that they comprise elongated portions 300 that extend from the rear wall 3b of the clamping member 101, 101' upwards. The elongated portions 300 have a width that is substantially the same as the width of the rear wall 3b in the region of the head receiving portion. A length of the elongated portions 300 is such that they can project out of the skin of a patient, when the distraction assembly has been mounted to the bone anchors. The elongated portions 300 may comprise a plurality of transverse perforations or slots 301. These slots 301 contribute to render the elongated portions 300 flexible. Moreover, the slots 300 may allow engagement with an instrument to spread them apart. Hence, the distraction assembly according to this embodiment may also be used as a retractor to keep the surgical site open. The clamping members 101, 101' may also be part of the modular system.

In a further modified embodiment, the distractor assembly according to Fig. 16 is used together with a spreader device 600 and a retractor system 800 comprising retractor blades 801. Arms 601 of the spreader device can engage the elongated portions 300 to spread them apart. In this case, the elongated portions 300 act as retractor blades. In addition, at least one retractor blade 801 of the retractor system can be mounted to the rod 100 and the opposite retractor blade 801 can then be used to provide a sufficient opening for the surgical operations, The distance of the retractor blades 801 from each other is adjustable.

Various modifications of the distraction and/or compression assembly may be contemplated. For example, the rod 100 does not have to be a separate part. It can be a projection integrally formed with one of the clamping members 1, 1'. Moreover, the rod 100 is not limited to a cylindrical rod. It may have any shape that allows to slide the clamping member thereon.

The clamping members can have any shape as long as they are configured to allow a pivoting of the head in a limited range of directions. In particular, the clamping elements can be formed otherwise as long as they are configured to exert pressure to the head and/or the neck or shank only from one side.

## Claims

1. Distraction and/or compression assembly, in particular for use in bone surgery, including
a first clamping member (1, 101) configured to engage a first bone anchor (10), wherein the first bone anchor comprises a head (12) and a neck (13) adjacent to the head (12),
a second clamping member (1', 101') configured to engage a second bone anchor (10'), wherein the second bone anchor comprises a head (12) and a neck (13) adjacent to the head (12),
a connection member (100) configured to connect the first clamping member (1, 101) and the second clamping member (1', 101');
wherein at least the first clamping member (1, 101) comprises a clamping element (20, 20') that can assume a clamping position in which the clamping element (20, 20') is configured to laterally enclose an inserted head (12) and to exert pressure onto the neck (13) from one side.

2. The assembly of claim 1, wherein the first clamping member (1, 101) comprises a seat (4) for an inserted head (12) and wherein the clamping element (20, 20') is configured to clamp an inserted head (12) in such a manner that the head (12) can pivot in the seat (4) in a direction away from the clamping element (20, 20').

3. The assembly of claim 1 or 2, wherein the clamping element (20, 20') is rotatably supported in the first clamping member (1, 101), preferably with an axis of rotation extending perpendicular to a longitudinal axis of the connection member (100).

4. The assembly of one of claims 1 to 3, wherein the clamping element (20, 20') has a hook-like shape the with a free end (20b) that is configured to engage the head (12) at a lower side of the head (12) or to engage the neck (13) of the bone anchor adjacent to a lower side of the head (12).

5. The assembly of one of claims 1 to 4, wherein the clamping member (1, 101) comprises a head receiving portion (2) comprising the seat (4) and a rod receiving portion (8), preferably wherein the rod receiving portion (8) is arranged laterally offset with respect to a center axis of the head receiving portion (2).

6. The assembly of one of claims 1 to 5, wherein the first clamping member (1, 101) comprises a head locking member (40) for clamping the head (12) of the bone anchor with respect to the first clamping member (1, 101).

7. The assembly of one of claims 1 to 6, wherein the connection member (100) is a rod that guided in a rod recess (9) in the first clamping member (9a).

8. The assembly of claim 7, wherein the rod recess (9) is formed as a circumferentially closed channel.

9. The assembly of one of claims 7 or 8, wherein the first clamping member (1, 101) comprises a rod locking member (30) for locking the first clamping member (1, 101) to the rod (100), preferably wherein the rod locking member (30) is configured to lock the first clamping member (1, 101) to the rod (100) independently from the head (12).

10. The assembly of one of claims 1 to 9, wherein the first connection member (1, 101) comprises extended portions (300) that are preferably flexible and preferably configured to act as retractor blades.

11. The assembly of one of claims 1 to 10, further comprising a first bone anchor (10) and a second bone anchor (10'), wherein the first bone anchor (10) and the second bone anchor (10') each comprise an anchoring section (11) configured to be anchored in bone, a head (12) and a neck (13) adjacent to the head (12) and wherein at least the head (12) of the first bone anchor (10) comprises a spherical outer surface portion.

12. The assembly of claim one of claims 1 to 11, wherein the connection member (100) and the first clamping member (1, 101) and the second clamping member (1', 101') are pre-assembled, preferably in such a manner that the first clamping member (1, 101) is movable along the connection member (100) and the second clamping member (1', 101') is fixed to the connection member (100).

13. The assembly of one of claims 1 to 12, wherein the second clamping member (1', 101') is shaped mirror-symmetrical to the first clamping member (1, 101).

14. The assembly of claim 13, wherein the assembly forms a distraction assembly when the first clamping member (1, 101) and the second clamping member (1', 101') are configured and arranged such that pressure is exerted onto the neck (13) by the clamping element (20, 20') from a side of the head (12) facing towards the respective other clamping member and wherein the assembly forms a compression assembly when the first clamping member (1, 101) and the second clamping member (1', 101') are configured and arranged such that pressure is exerted onto the neck (13) by the clamping element (20, 20') from a side of the head (12) facing away from the respective other clamping member.

15. The assembly of one of claims 1 to 14, further comprising distraction/ compression pliers (200) having a front end (210, 220) configured to be placed onto the connection member (100).
